# EUROPÄISCHE PATENTSCHRIFT

(11) **EP 2 204 095 B1**
(45) Veröffentlichungstag und Bekanntmachung des Hinweises auf die Patenterteilung: **13.04.2011**
(21) Anmeldenummer: 08172799.2
(22) Anmeldetag: 23.12.2008
(51) Int. Cl.: A21D 2/14, A21D 2/18, A21D 2/36, A21D 10/00

(54) **Mischkristalle, Verfahren zu deren Herstellung und Verwendung in der Herstellung von Backwaren**
Mix crystal, method for their manufacture and application thereof in the production of baking goods
Cristaux mélangés, leur procédé de fabrication et d'utilisation dans la fabrication de produits cuits

(43) Veröffentlichungstag der Anmeldung: 07.07.2010
(73) Patentinhaber: BASF SE, 67056 Ludwigshafen (DE)
(72) Erfinder: Diener, Ralf, 67435, Neustadt (DE); Schneider, Jürgen, 67273, Bobenheim am Berg (DE); Seiter, Herbert, 66996, Schindhard (DE); Schäfer, Manfred, 67117, Limburgerhof (DE); Schmid, Walther, 68161, Mannheim (DE)

(56) Entgegenhaltungen:
- EP-A- 1 161 872
- EP-A- 1 260 147
- WO-A-01/05246
- WO-A-94/24994
- WO-A-97/12607
- WO-A-2004/048418
- US-A- 1 643 951
- US-A- 2 791 508
- US-A- 3 930 032
- US-A1- 2005 118 326
- US-A1- 2005 233 046

## Beschreibung

Die vorliegende Erfindung betrifft Mischkristalle enthaltend a) Backtriebmittel und b) 0,1 bis 5000 Gew.-ppm Antibackmittel, bezogen auf die Gesamtmenge des Backtriebmittels, in Form von mindestens einem Polymer, wobei beim Einsatz von hydrophilen Cellulosederivaten als Antibackmittel sich deren Menge auf kleiner 100 Gew.-ppm, bezogen auf die Gesamtmenge des Backtriebmittels, reduziert.
Ferner betrifft die vorliegende Erfindung die Herstellung der Mischkristalle und deren Verwendung in der Herstellung von Backwaren, als Säureregulator in Speisen, in der Herstellung von Kosmetikprodukten, in der Synthese und Formulierung von Pharmaprodukten, sowie als Treibmittel in technischen Verfahren wie z. B. die Herstellung von Moosgummi oder für Feuerlöschformulierungen.
Darüber hinaus betrifft die vorliegende Erfindung die Herstellung von Backwaren.

Zur Herstellung von porösen Backwaren wird im Teig vor und/oder während des Backvorgangs ein Gas erzeugt oder der Teig mit Gas versetzt, um im fertigen Backwerk durch die Gasblasen Porosität zu erzeugen. Im einfachsten Fall wird der Teig vor dem Backen mit einem Gas, meist Luft, versetzt, etwa durch intensives Schlagen des Teigs oder eines seiner Bestandteile vor dem Vermischen. Die bekannteste Ausführungsform ist die Zugabe von Eischnee zum Teig. Es ist genauso möglich, ein Gas wie Luft durch Düsen in den Teig einzubringen. Ebenso bekannt sind Verfahren, bei denen Wasserdampf den Teig lockert, beispielsweise bei der Herstellung von Blätterteig. Das meist verwendete Gas ist jedoch Kohlendioxid oder Kohlendioxid im Gemisch mit Ammoniak und Wasserdampf. Kohlendioxid wird beispielsweise biologisch im Zuge der Vergärung von Bestandteilen des Teigs durch Hefen (Hefeteig) und/oder Milchsäurebakterien (im so genannten Sauerteig) erzeugt. Alternativ oder zusätzlich zur Verwendung von Hefe oder Sauerteig wird Kohlendioxid, oder das Kohlendioxid-, Ammoniak- und Wasserdampfgemisch auch chemisch durch Backzusätze, so genannte Backtriebmittel oder Umgangssprachlich "Backpulver", erzeugt, die dem Teig zugesetzt werden.

Backtriebmittel enthalten im Allgemeinen mindestens ein Carbonat und, falls sich dieses nicht allein durch Temperaturerhöhung zersetzt, eine saure oder säurebildende Substanz. Sie enthalten wahlweise neben Carbonat auch Carbamat (alter Name: "Carbaminat"). Das Carbonat und/oder Carbamat wird dem herzustellenden Backwerk entsprechend gewählt, beispielsweise wird für Leb- oder Honigkuchen häufig Kaliumcarbonat verwendet, für Flachbackwaren Natriumhydrogencarbonat (alter Name: "Natriumbicarbonat"), Ammoniumhydrogencarbonat ("Ammoniumbicarbonat", abgekürzt "ABC"), als alleiniges Carbonat oder im Gemisch mit Ammoniumcarbamat ("Ammoniumcarbonat"). Die Säure oder der Säurebildner darf- auch zusammen mit den sich nicht verflüchtigenden Resten des Carbonats oder Carbamats - den Geschmack nicht negativ beeinflussen. Typischerweise werden Verbindungen wie Weinsäure oder ihre Salze, etwa Kalium-, Natrium-, Kaliumhydrogen- und/oder Calciumtartrat, Citronensäure, Calciumhydrogenphosphat, Natriumhydrogenpyrophosphat oder Natriumaluminiumphosphat verwendet. Wenn das Backtriebmittel eine Säure oder einen Säurebildner enthält, wird ihm meist ein Trennmittel zugegeben, das die vorzeitige Kohlendioxidbildung durch Reaktion des Carbonats mit der Säure oder dem Säurebildner verhindert, üblich ist hierfür der Zusatz von Mehl oder Stärke. Die genannten Ammoniumverbindungen ABC und Ammoniumcarbaminat zersetzen sich allein durch Temperaturerhöhung auf mindestens 60°C rückstandsfrei in Kohlendioxid, Ammoniak und Wasser. Bei typischen Backtemperaturen fallen alle drei Komponenten gasförmig an und führen daher sämtlich zu einer Erhöhung der Porosität des Backwerks. Diese Verbindungen werden daher typischerweise ohne Zusatz einer Säure oder eines Säurebildners verwendet, so dass sich der Zusatz des Trennmittels erübrigt.

Ullmanns Encyklopädie der technischen Chemie, 3. Auflage, Urban & Schwarzenhäuser, München - Berlin 1953, Stichwort "Backpulver" oder Ullmann's Encyclopedia of Industrial Chemistry, Sixth Ed., 1999 Electronic Release, Wiley-VCH, Weinheim 1999, Stichwort "Bread and other baked Products", dort Punkt 2.6: "Leavening Agents", geben einen zusammenfassenden Überblick über die bekannten Verfahren zur Herstellung poröser Backwaren unter Verwendung von Backtriebmitteln. Die Herstellung von Ammoniumverbindungen wie Ammoniumcarbonat, -bicarbonat und -carbaminat durch Umsetzung von dem gewünschten Produkt entsprechenden Mengen Ammoniak und Kohlendioxid in wässriger Mutterlauge, bei dem Produkt entsprechend gewählten Drücken und Temperaturen, gefolgt von Abtrennung und Trocknung des Niederschlags, ist ebenso seit langem bekannt und beispielsweise in Ullmann's Encyclopedia of Industrial Chemistry, Sixth Ed., 1999 Electronic Release, Wiley-VCH, Weinheim 1999, Stichwort "Ammonium Compounds", insbesondere Punkt 4.: "Ammonium Carbonates", beschrieben. Die Herstellung der Alkalicarbonate und -hydrogencarbonate ist ebenso bekannt.

Obwohl bei typischen Backtemperaturen diese Ammoniumverbindungen gasförmig anfallen, wird häufig ein Antibackmittel zugesetzt, um das als "Verbacken", englisch "caking", bezeichnete Entstehen von Klumpen oder größeren Agglomeraten in einem Pulver zu verhindern (der Begriff "Verbacken" oder "Antibackmittel" hat außer seinem Wortstamm nichts mit der Herstellung von Backwaren, Gebäck oder dem Begriff "Backtriebmittel" für Stoffe, die zu Porosität im Backwerk führen, zu tun). Als Antibackmittel werden hierfür üblicherweise Maismehl, Magnesiumoxid oder Magnesiumcarbonat in einer Menge von 2000 bis 10000 Gew.-ppm verwendet. Gegebenenfalls werden ferner anorganische Salze mit den Antibackmitteln vermischt.

EP-A 1 161 872 offenbart den Einsatz von Antibackmitteln auf Basis von hydrophilen Cellulosederivaten. Als bevorzugte hydrophile polymere Cellulosederivate werden Natriumcarboxymethylcellulose genannt. Es wird offenbart, dass das Cellulosederivat in einer Menge von mindestens 100 Gew.-ppm, bezogen auf die Gesamtmenge des Backtriebmittels, besonders bevorzugt mindestens 500 Gew.-ppm, beispielsweise mit 1000 Gew.-ppm, eingesetzt wird.

US-A 3 930 032 offenbart ein modifiziertes Backtriebmittel das stabiler ist, weil Natriumbicarbonat mit Cellulosederivaten überzogen ist. Dieses bedecktes Backtriebmittel enthalt 3% von Cellulosederivaten.

Hydrophile Cellulosederivate finden sich in Backwaren nicht nur als Antibackmittel, sondern werden ferner als Ballaststoffe in einer Menge von typischerweise 5 bis 20 Gew.-% bezogen auf die Backware verwendet (US 4,678,672 und GB 745,926). Ferner ist die Verwendung von Carboxymethylcellulose als Verdickungsmittel in einer üblichen Menge von 0,5 bis 1 Gew.-% bezogen auf die Backware bekannt (EP-A 399 995).

Der Einsatz von Antibackmitteln ist des weiteren bei Düngemitteln weit verbreitet, da die Düngemittel aufgrund der nur saisonalen Verwendung vergleichsweise lagerstabil sein müssen.

Als Antibackmittel werden beispielsweise Leime auf Basis von Carboxymethylcellulose, die mit Füllstoffen wie Calciumcarbonat oder Calciumoxid vermischt sind (DD-A 117 787), synthetische Polymere wie beispielsweise Carboxymethylcellulose oder Methylcellulose und eine oberflächenaktive Substanz (US 3,388,990/US 5,472,476), Hydroxypropylcellulose, Natriumcarboxymethylcellulose oder Hydroxypropylmethylcellulose (EP-A 246 719) oder Natriumcarboxylmethylcellulose und eine oberflächenaktive Substanz (SU-A 1570255) verwendet.

Im Stand der Technik wird ferner offenbart, die anorganischen Salze mit einem Schutzfilm zu überziehen. Als Schutzfilm werden beispielsweise Polysaccharide und ihre Derivate beschrieben, insbesondere Stärke, Cellulose, Manna, Natriumalginat, Methylcellulose, Carboxymethylstärke und Carboxymethylcellulose (DE-A 28 21 703, WO 98/56595). Ferner werden zum Überziehen von Partikeln wasserlösliche Celluloseester (EP-A 461 886) oder filmbildenden Polymere wie Methylcellulose, Hydroxibutylmethylcellulose, Natriumcarboximethylcellulose, Hydroxiethylmethylcellulose oder Hydroxipropylmethylcellulose (DE-A 24 35 008) eingesetzt. Gegebenenfalls können weitere Antibackmittel wie Magnisiumsilikat (WO 94/24860, WO 94/24994, US 5,482,702) oder Phosphorverbindungen (US 5,468,716) eingesetzt werden.

Nachteilig am Stand der Technik ist, dass bei den bekannten Backverfahren unter Verwendung üblicher Backtriebmittel die Bildung von unerwünscht großen Gasblasen und in Folge davon unerwünscht großen Hohlräume im Backwerk trotz Verwendung von Antibackmitteln nicht gänzlich unterdrückt werden kann. Diese großen Hohlräume gehen häufig weit über den gewünschten Porendurchmesser von 0,1 - ca. 5 mm der Poren im Backwerk hinaus. Dies führt zu einem unerwünscht hohen Anteil an Ausschuss, da solches Backwerk zwar einzelne sehr große Hohlräume, aber ansonsten überwiegend Bereiche mit unerwünscht niedriger Porosität enthält, was das Backwerk hart und häufig auch unansehnlich macht. Wenn die großen Hohlräume an der Oberfläche des Backwerkes auftreten so wird beim Backprozess die dann dünne Teigschicht on der Oberseite wesentlich schneller fertig gebacken und weist dann eine unschöne dunkelbraune bis schwarze Verfärbung auf. Diese Backwaren sind dann nicht verkaufsfähig und erhöhen den Ausschuss bei der Produktion.

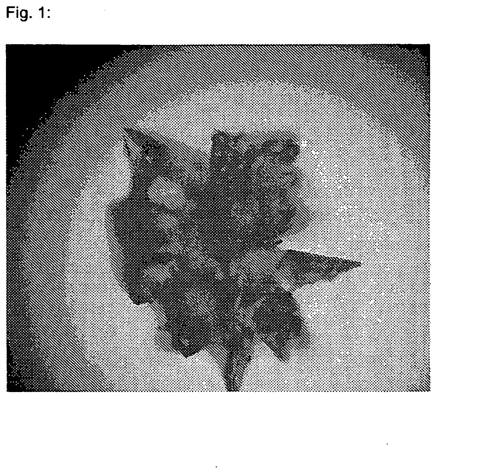

Das Verbacken der gängigen Backtriebmittel beruht unter anderem auf der Kristallform der Produkte. Die technisch gut verfügbaren Salze weisen durch Aufwachsungen und andere Kristallfehler eine sehr raue Struktur auf. Diese Kristallformen können aufgrund der rauen Struktur sehr leicht verhaken und es bilden sich mechanisch stabile Brücken zwischen den einzelnen Kristallen. Diese stabilen Brücken bewirken die bekannt hohe Verbackungsneigung der Produkte. In der Figur 1 ist ein Ammoniumbicarbonat-Agglomerat abgebildet, das üblicherweise im Stand der Technik als Backtriebmittel eingesetzt wird.

Im Stand der Technik wird demnach Antibackmittel im Backtriebmittel eingesetzt, um das Verhaken und somit Agglomerieren der Backtriebmittelkristalle zu reduzieren. Das Antibackmittel wird folglich als Abstandshalten oder zum Beschichten/Coaten der rauen Kristallstruktur (EP-A 1 161 872), um diese abzurunden, eingesetzt.

Ferner sind diese Mischung aus Backtriebmittel und Antibackmittel beim Einsatz im kontinuierlichen Prozess zur Herstellung von Backwaren (automatisierte Backstraßen) nachteilig. Typischerweise werden diese Mischungen in Pulverform über eine Dosiervorrichtung eingebracht. Um ein Verbacken der Mischung in der Dosiervorrichtung zu verhindern, wird diese ständig gerüttelt. Das Rütteln bewirkt aber leider eine Separation des Backtriebmittels und des Antibackmittels. Folglich liegt das Antibackmittel nicht mehr gleichmäßig im Backtriebmittel vor und es kann zum Auftreten von Verbackungen und somit zur unerwünschten Bildung von ungleichmäßig großen Hohlräumen im Backwerk und folglich zu vermehrten Ausschuss kommen.

Ein weiterer Nachteil der Backtriebmittel aus dem Stand der Technik stellt deren geringe Lagerfähigkeit dar. Insbesondere bei der im Anwendungsgebiet üblichen Lagerung unter Druck werden im Stand der Technik schon nach wenigen Tagen Verbackungen sowohl bei den gecoateten Backtriebmitteln als auch bei der reinen pulvrigen Mischung aus Backtriebmittel und Antibackmittel festgestellt. Eine Lagerung unter Druck ist üblich, da aus Platzmangel die jeweiligen Säcke übereinander gelagert werden.

Folglich stellen trotz Verwendung von Antibackmitteln Verbackungen, d.h. das Entstehen und Klumpen oder größeren Agglomeraten, bei der Lagerung und dem Einsatz von Backtriebmitteln immer noch ein ungelöstes Problem dar. Die Verbackungen bewirken die Bildung von unerwünscht großen Hohlräumen und somit eine ungleichmäßige Verteilungen von Hohlräumen.

Die Aufgabe der vorliegenden Erfindung ist demnach, ein modifiziertes Backtriebmittel bereitzustellen, das im Vergleich zum Stand der Technik weniger leicht agglomeriert und/oder deren Agglomerate sich einfach wieder auflockern lassen. Ferner soll im Vergleich zum Stand der Technik erstmals die Ursache des Agglomerisierens, d.h. die Kristallform des Backtriebmittels modifiziert werden, anstatt lediglich die Auswirkung der Kristallform zu minimieren. Darüber hinaus soll dieses modifizierte Backtriebmittel trotz Einsatz nur sehr niedriger Mengen an Hilfsmittel auch bei langer Lagerung noch ein freifließendes Pulver bilden, das bei Verwendung in automatiserten/kontinuierlichen Backstraßen genau dosierbar bleibt. Insbesondere soll bei der Herstellung von Backwaren die Bildung von unerwünscht großen Hohlräumen vermieden werden.

Überraschenderweise wurde gefunden, dass Mischkristalle enthaltend
a) Backtriebmittel
b) 0,1 bis 5000 Gew.-ppm Kristallisationshilfsmittel, bezogen auf die Gesamtmenge des Backtriebmittels, in Form von mindestens einem Polymer, wobei auch Mischungen verschiedener Polymere verwendbar sind,
   wobei beim Einsatz von hydrophilen Cellulosederivaten als Kristallisationshilfsmittel sich deren Menge auf kleiner 100 Gew.- ppm, bezogen auf die Gesamtmenge des Backtriebmittels reduziert,
eine gleichmäßige und nichtverbackende Kristallstruktur aufweisen.

Unter dem Begriff Mischkristall wird bei der vorliegenden Erfindung verstanden, dass das Kristallisationshilfsmittel (Komponente b) im und/oder auf dem Backtriebmittelkristall (Komponente a), d.h. im Kristall und/oder auf/an der Kristalloberfläche, vorliegt.

Die Mischkristalle stellen folglich ein modifiziertes Backtriebmittel dar.

Unter dem Begriff "rieselfähig" wird bei der vorliegenden Erfindung verstanden, dass das Produkt nach z.B. einer Lagerung oder einem Transport in Säcken nach deren Öffnen nicht in Form von einem oder mehreren Brocken heraus fällt, d.h. stark verklumpt ist, sondern frei aus dem Sack ausfließt / herausrieselt.

Unter dem Begriff "klumpenfrei" wird bei der vorliegenden Erfindung verstanden, dass das rieselfähige Pulver frei von Agglomeraten, die sich während der Lagerung gebildet haben, ist.

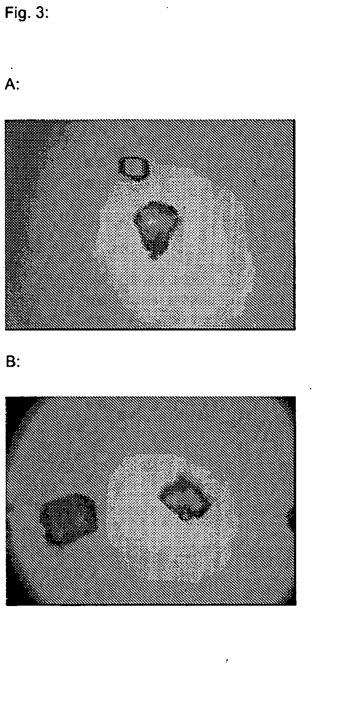

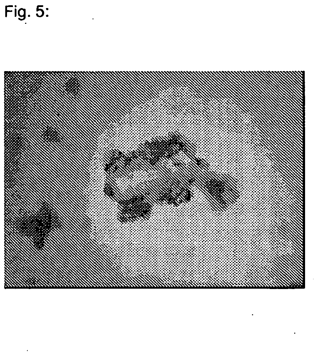

Die erfindungsgemäßen Mischkristalle weisen eine gleichmäßige, runde, glatte und somit nichtverbackende Kristallstruktur auf (siehe Figuren 3 A/B, 4 A/B und 5).

Es bilden sich bei der Lagerung und bei der Anwendung wesentlich weniger und darüber hinaus leichter wieder auflockerbare Agglomerate und Verbackungen als bei Backtriebmitteln aus dem Stand der Technik.

Das Backtriebmittel (Komponente a) enthält mindestens ein Carbonat. Als Carbonat wird oder werden solche Carbonate gewählt, deren Verwendung in Nahrungsmitteln unbedenklich ist und die ebenso wie ihre Zersetzungsprodukte nicht zu unangenehmen Geschmack der fertigen Backwaren führen. Geeignete Carbonate, die einzeln oder im Gemisch enthalten sind, sind dem Fachmann bekannt und typischerweise werden Alkalicarbonate und -hydrogencarbonate, insbesondere Natriumcarbonat, Natriumhydrogencarbonat, Kaliumcarbonat und Kaliumhydrogencarbonat sowie Ammoniumcarbonat und Ammoniumhydrogencarbonat verwendet.

Ebenso geeignet ist das üblicherweise als "Hirschhornsalz" bezeichnete Gemisch aus Ammoniumcarbonat und Ammoniumhydrogencarbonat, das zusätzlich auch Ammoniumcarbamat enthalten kann.

Vorzugsweise ist das Carbonat Ammoniumhydrogencarbonat und/oder Ammoniumcarbonat . Besonders bevorzugt ist das Carbonat Ammoniumhydrogencarbonat (auch Ammoniumbicarbonat genannt).

Die mittleren Partikeldurchmesser der eingesetzten Carbonate liegen im allgemeinen bei 50 bis 1000 µm, vorzugsweise bei 75 bis 700 µm, bevorzugt bei 150 bis 500 µm.

Wahlweise enthält das Backtriebmittel zusätzlich ein oder mehrere Carbamat(e). Als Carbamat wird oder werden solche Carbamate gewählt, deren Verwendung in Nahrungsmitteln unbedenklich ist und die ebenso wie ihre Zersetzungsprodukte nicht zu unangenehmen Geschmack der fertigen Backwaren führen. Ein geeignetes Carbamat ist beispielsweise Ammoniumcarbamat.

Falls das Backtriebmittel Carbamat enthält, beträgt die Menge an Carbamat vorzugsweise 10 bis 90 Gew.-%, bezogen auf die Gesamtmenge des Backtriebmittels, bevorzugt 30 bis 70 Gew.-%, insbesondere ca. 50% Gew.-%. Das Gemisch aus gleichen Teilen Ammoniumcarbamat und Ammoniumbicarbonat wird auch als Ammoniumcarbonat bezeichnet.

Wenn das eingesetzte Backtriebmittel Komponenten enthält, die sich beim Erwärmen auf typische Backtemperaturen von beispielsweise 100 bis 200 °C nicht oder nicht ausreichend zersetzen, enthält das Backtriebmittel zusätzlich eine Säure oder einen Säurebildner. Die Säure oder der Säurebildner ist eine für diesen Verwendungszweck bekannte Verbindung oder Verbindungsgemisch, beispielsweise Kalium-, Natrium-, Kaliumhydrogen- und/oder Calciumtartrat, Citronensäure, Calciumhydrogenphosphat, Natriumhydrogenpyrophosphat und/oder Natriumaluminiumphosphat. Falls das Backtriebmittel Säure oder Säurebildner enthält, beträgt die Menge an Säure oder Säurebildner vorzugsweise so viel, wie zur Umsetzung des Backtriebmittels und damit zur Freisetzung von Kohlendioxid benötigt wird. Je nach Säurestärke, Anzahl der Protonen pro Molekül und Molgewicht der Säure und des Backtriebmittels kann diese Menge stark unterschiedlich sein. Als Beispiel gilt bei Verwendung von Natriumbicarbonat und für gebräuchliche Säureträger ein Bereich von 60 bis 250 Gew.-%, bezogen auf die Gesamtmenge des Backtriebmittels, bevorzugt 75 bis 225 Gew.-%.

Wenn das Backtriebmittel eine Säure oder einen Säurebildner enthält, wird ihm vorzugsweise auch ein Trennmittel zugegeben, das die vorzeitige Kohlendioxidbildung durch Reaktion des Carbonats mit der Säure oder dem Säurebildner verhindert. Derartige Trennmittel sind bekannt, bevorzugt sind Mehl und/oder Stärke.

Die mittleren Partikeldurchmesser der eingesetzten Säuren oder Säurebilder liegen im allgemeinen bei 50 bis 1000 µm, vorzugsweise bei 75 bis 700 µm, bevorzugt bei 150 bis 500 µm.

Die genannten Carbonate, Carbamate, Säuren oder Säurenbildner, sowie Trennmittel sind kommerziell erhältlich.

Das Kristallisationshilfsmittel (Komponente b) beinhaltet bevorzugt mindestens ein kristallisationsbeeinflussendes Polymer. Unter dem Begriff "kristallisationsbeeinflussend" wird in der vorliegenden Erfindung verstanden, dass das zugesetzte Polymer die Kristallform der entstehenden Krisalle in geeigneter Art und Weise beeinflußt. Unter der Einwirkung der kristallisationsbeeinflussenden Polymere weisen die Kristalle eine glatte und regelmäßige Struktur auf. Diese Struktur steht im Gegensatz zur Kristallstruktur ohne kristallisationsbeeinflussendes Polymer, die durch störende Aufwachsungen und andere Kristallformen mit unregelmäßiger und rauer Oberfläche gekennzeichnet ist.

Ferner ist das Polymer bevorzugt hydrophil. Es können ungeladene, anionische und/oder kationische kristallisationsbeeinflussende Polymere verwendet werden.

Ferner können neben einzelnen Polymeren auch Mischungen aus verschiedenen nichtgeladenen, verschiedenen anionischen und/oder verschiedenen kationischen Polymeren eingesetzt werden. Daneben können auch nichtgeladene Polymere als Mischungskomponenten für anionische und/oder kationische Polymermischunge verwendet werden.

Diese Polymere werden aus offensichtlichen Gründen vorteilhaft so gewählt, dass sowohl das Polymer selbst wie auch etwaige thermische Abbauprodukte in den typischerweise enthaltenen oder entstehenden Mengen als Lebensmittelzusatzstoff geeignet sind und die hergestellten Backwaren geschmacklich nicht beeinträchtigen. Vorzugsweise werden Polymere natürlichen Ursprungs oder solche, die durch Modifikation natürlicher Polymere entstehen, verwendet, die geschmacksneutral und lebensmittelrechtlich zugelassen sind.

Als geladene natürliche Polymere sind hydrophile Polymere auf Basis von Zuckerderivaten und/oder Peptiden bevorzugt, insbesondere aus den Bereichen der (Hetero)Polysaccharide und/oder (Poly)Peptide.

Diese Heteropolysaccharide werden üblicherweise durch Fermentation oder durch Isolierung aus natürlichen Quellen gewonnen.

Besonders bevorzugt sind geladene (Hetero)Polysaccharide mit Carboxyl- oder Sulfonat-Seitenketten, insbesondere mit nichtmodifizierten Carboxylgruppen (z. B. Pektin und/oder Alginat) und mit modifizierten Carboxylgruppen (z. B. amidiertes Pektin).

Vorteilhafte anionische Polymere stellen beispielsweise Polyacrylsäure und deren Salze mit Ammonium, Natrium oder Kalium, Polymethacylsäure und deren Salze mit Ammonium, Natrium oder Kalium, Acrylsäure und deren Salze mit Ammonium, Natrium oder Kalium, Methacrylsäure-Copolymere und deren Salze Ammonium, Natrium oder Kalium, Acrylsäure-Maleinsäure-Copolymere und deren Salze mit Ammonium, Natrium oder Kalium dar.

Die genannten anionischen Polymere können ferner Vinylsulfonsäure in veränderlichen Anteilen enthalten.

Alle genannten anionischen Polymere können teilveresterte Gruppen aufweisen, wobei als Alkoholkomponenten sowohl aliphatische als auch derivatisierte Komponenten auf Basis von endgruppenverschlossenen Polyalkylenglykole sein können. Für die Herstellung der Polyalkylenglykole können sowohl Ethylenoxid als auch Propylenoxid und höhere Alkylenoxide allein oder in Form von Random oder Blockpolymeren verwendet werden.

Ferner ist Polyasparaginsäure und deren Salze mit mono- und divalenten Kationen bevorzugt. Besonders bevorzugt sind die Salze der Polyasparaginsäure mit Ammonium, Natrium und Kalium.

Vorteilhafte kationische Polymere stellen beispielsweise Polymine, Polyvinylamine und Copolymere mit Polyvinylalkohol und/oder Poly-dimethyl-allyl-ammoniumchlorid dar.

Vorteilhafte nicht-ionische Polymere stellen beispielsweise Polyethylenglykole, Polypropylenglykole, Random und Blockpolymere auf Basis Ethylenoxid mit Alkylenoxiden, insbesondere Propylenoxid und/oder Butylenoxid dar. Gegebenenfalls weisen diese ferner einen zusätzlichen Endgruppenverschluss auf einer oder beiden Seiten mit aliphatischen Endgruppen auf. Ferner ist Polyvinylpyrrolidon und/oder Polyvinylpolypyrrolidon bevorzugt.

Insbesondere sind solche Polymere bevorzugt, die bereits eine Zulassung als Lebensmittelzusatzstoff aufweisen, wie beispielsweise: Alginsäure (E 400), Natriumalginat (E 401), Kaliumalginat (E 402), Ammoniumalginat (E 403), Calciumalginat (E 404), Propylenglycolalginat (E 405), Agar-Agar (E 406), Carrageen (E 407), Verarbeitete Eucheuma-Algen (E 407a), Johannisbrotkernmehl (E 410), Guarkernmehl (E 412), Traganth (E 413), Gummi arabicum (E414), Xanthan (E 415), Karaya (E 416), Tarakernmehl (E 417), Gellan (E 418), Komjak-Gummi/Konjak-glucomannan (E 425), Sojabohnen-Polyose (E426), Pektin/Amidiertes Pektin (E 440), Mikrokristalline Cellulose/cellulosepulver (E 460), Methylcellulose (E 461), Ethylcellulose (E 462), Hydroxypropylcellulose (E 463), Hydroxypropylmethylcellulose (E 464), Ethylmethylcellulose (E 465), Carboxymethylcellulose/Natriumcarboxymethylcellulose (E 466), Vernetzte Natriumcarboxymethylcellulose (E 468), enzymatisch hydrolysierte Carboxymethylcellulose (E 469), Polydextrose (E 1200), Polyvinylpyrrolidon (E 1201), Polyvinylpolypyrrolidon (E 1202), Pullulan (E 1204), Oxidierte Stärke (E 1404), Monostärkephosphat (E 1410), Distärkephosphat (E 1412), Phosphatiertes Distärkephosphat (E 1413), Acetyliertes Distärkephosphat (E 1414), Acetylierte Stärke (E 1420), Acetylierte Distärkeadipat (E 1422), Hydroxypropylstärke (E 1440), Hydroxypropyldistärkephosphat (E 1442), Stärkenatriumoctenylsuccinat (E 1450), Acetylierte oxydierte Stärke (E 1451).

Besonders bevorzugt sind Kristallisationshilfsmittel ausgewählt aus der Gruppe bestehend aus Alginsäure (E 400), Natriumalginat (E 401), Kaliumalginat (E 402), Ammoniumalginat (E 403), Calciumalginat (E 404), Propylenglycolalginat (E 405), Pektin (E 440), amidiertes Pektin (E 440), Carrageen (E 407), Gellan (E 418), Gummi Arabicum (E414), Karaya (E 416), Traganth (E 413) und/oder Xanthan (E 415), ganz besonders bevorzugt Pektin (E 440) und/oder amidiertes Pektin (E 440).

Ferner sind als Kristallisationshilfsmittel lineare und/oder verzweigte (Poly)Peptide bevorzugt. Besonders bevorzugt im Bereich der (Poly)Peptide sind Gelatinequalitäten.

Geeignete Cellulosederivate sind beispielsweise Celluloseether. Dies sind Cellulosederivate, die formal durch Substitution von Wasserstoffatomen an den Hydroxigruppen der Cellulose durch Alkyl- und/oder Arylalkylgrupen hervorgehen, wobei diese Alkylund/oder Arylalkylgruppen durch funktionelle nichtionische, anionische und/oder kationische Gruppen substituiert sein können. Die Alkylgruppen sind üblicherweise C1- C8 Alkylgruppen, die geradkettig oder verzweigt sein können. Vorzugsweise ist die Alkylgruppe eine C1-C4-Alkylgruppe, beispielsweise Methyl, Ethyl, n-Propyl, iso-Propyl, n-Butyl, sec.-Butyl, iso-Butyl oder tert.-Butyl. Die Alkylgruppe kann mit einem aromatischen Rest zur Arylalkylgruppe substituiert sein, beispielsweise mit einem Phenylrest. Eine bevorzugte Arylalkylgruppe ist Benzyl. Die Alkyl- oder Arylalkylgruppe kann funktionell substituiert sein, beispielsweise mit Hydroxi-, Carboxi- oder Carboxilatgruppen. Sofern Carboxilatgruppen enthalten sind, sind auch entsprechende Gegenionen enthalten, beispielsweise Alkaliionen wie Natrium oder Kalium, oder Ammoniumionen. Ist nur von "Carboximethylcellulose" (oft als "CMC" abgekürzt) die Rede, ist üblicherweise Natrium-carboximethylcellulose gemeint (gelegentlich auch als "Na-CMC" abgekürzt). Es können auch Cellulose-mischether verwendet werden, die mehr als eine Art Alkyl-Arylalkyl- oder funktionell substitutierte Alkylgruppe enthalten.

Bevorzugte hydrophile polymere Cellulosederivate sind Methyl- Ethyl-, Propyl-, Carboximethyl- Hydroxiethyl-, Hydroxipropyl- Methylhydroxidethyl-, Methylhydroxipropyl-, Methylhydroxibutyl-, Ethylhydroxidethyl-, Carboximethylhydroxiethyl- und/oder Benzylcellulose. Unter den Carboximethylcellulosen ist die Natriumverbindung bevorzugt. In bevorzugter Weise enthält das Backtriebmittel Natriumcarboximethylcellulose.

Die Celluloseether werden auf bekanntem Wege hergestellt, typischerweise durch Einwirkung von Alkyl- oder Arylalkylhalogeniden, Epoxiden oder aktivierten Olefinen auf mit Basen (beispielsweise Natronlauge) aktivierte Cellulose. Celluloseether sind gängige Handelswaren, die - auch in Lebensmitteln - üblicherweise als Verdickungsmittel verwendet werden. Celluloseether sind beispielsweise unter der Marke "Tylose", hochreine Celluloseether für Lebensmittelanwendungen unter der Marke "Tylopur", hochreine Na-CMC unter der Marke "Tylopur C" von Clariant GmbH erhältlich.

Als Gegenionen kommen neben Wasserstoff auch Alkali- und/oder Erdalkali-lonen, sowie substituierte oder unsubstituierte Amine, beispielsweise Ammoniak, in Frage.

Die Herstellung der genannten Polymere ist allgemein bekannt und in gängiger Fachliteratur nachzulesen.

Die Mischkristalle enthalten das Kristallisationshilfsmittel vorzugsweise in einer Menge von 0,5 bis 5000 Gew.-ppm, bezogen auf das Backtriebmittel, bevorzugt von 0,5 bis 2000 Gew.-ppm, besonders bevorzugt von 1 bis 1000 Gew.-ppm, insbesondere von 1 bis 500 Gew.-ppm, ganz besonders bevorzugt von 1 bis 100 Gew.-ppm, darüber hinaus bevorzugt von 1 bis 50 Gew.-ppm, des weiteren bevorzugt von 1 bis 20 Gew.-ppm.

Falls hydrophile Cellulosederivate als Kristallisationshilfsmittel eingesetzt werden, beträgt die Menge an hydrophilen Cellulosederivaten im Backtriebmittel vorzugsweise weniger als 80 Gew.-ppm, bezogen auf das Backtriebmittel, bevorzugt zwischen 0,5 und 50 Gew.-ppm, insbesondere zwischen 1 und 20 Gew.-ppm.

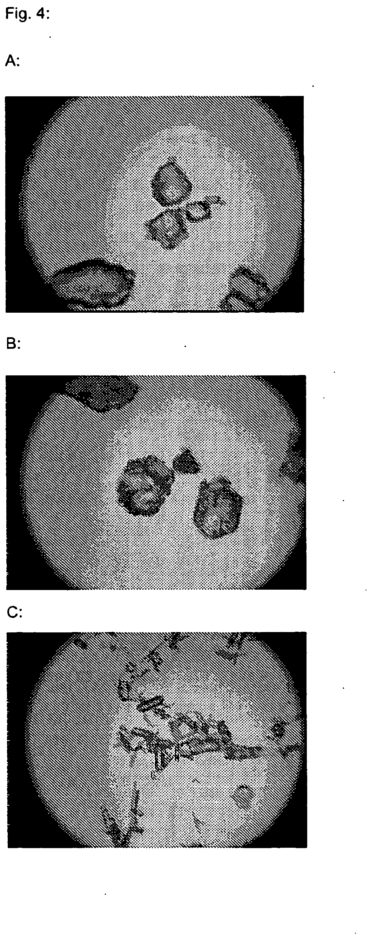

Die Steigerung der Menge an Kristallisationshilfsmittel über einen Höchstwert führt zu negativen Einflüssen auf die gewünschte Kristallmodifizierung und Kristallisationsneigung. Im Fall des Einsatzes von hydrophilen Cellulosederivaten als Kristallisationshilfsmittel werden bereits ab einem Wert von ca. 100 Gew.-ppm unerwünschte Ausbildungen von Stabkristallen anstatt kugelförmigen Kristallen erhalten (siehe Figur 4 C).

Diese Stabkristalle neigen wiederum zum Verhaken und somit zum Verbacken, ähnlich wie das Ammoniumbicarbonat aus dem Stand der Technik (siehe Figur 1). Zusätzlich werden sie nach einiger Zeit feucht und bilden dann eine klebrige Masse.
Unter Verwendung von mehr als 5000 Gew.-ppm Kristallisationshilfsmittel werden überwiegend sehr große Kristalle und/oder Kristalle mit raueren Oberflächen erhalten. Die großen Kristalle müssten dann vor einem Einsatz vermahlen werden. Das Vermahlen würde einen zusätzlichen, kostenintensiven Verfahrensschritt bedeuten, der ferner die Gefahr der Herstellung von Teilchen mit rauen Oberflächen in sich trägt.

Ferner betrifft die vorliegende Erfindung das Verfahren zur Herstellung der erfindungsgemäßen Mischkristalle, das dadurch gekennzeichnet ist, dass das Kristallisationshilfsmittel vor und/oder während des Kristallisationsschritts des Backtriebmittels zugegeben wird.

Bevorzugt wird das Kristallisationshilfsmittel der Mutterlauge, aus der das Backtriebmittel auskristallisiert wird, zugegeben. Hierbei wird das Kristallisationshilfsmittel der üblicherweise im Kreis gefahrenen Mutterlauge, in der das Carbonat und gegebenenfalls Hydrogencarbonat und Carbamat hergestellt und auskristallisiert wird, zugesetzt.

Das Verfahren zur Herstellung der Backtriebmittel ist dem Fachmann seit langem bekannt. Beispielsweise werden Ammoniumverbindungen wie Ammoniumcarbonat, - bicarbonat und -carbaminat durch Umsetzung von den entsprechenden Mengen Ammoniak, typischerweise 10 bis 20 %, und im Überschuss zugegebenen Kohlendioxid, typischerweise 30 bis 65 % in wässriger Mutterlauge, bei dem entsprechenden Druck, typischerweise 1 bis 6 bar und Temperatur, typischerweise 30 bis 65 °C gefolgt von Kristallisation, Abtrennung und Trocknung des Niederschlags hergestellt.

Eine detaillierte Beschreibung der Herstellung von Backtriebmitteln findet sich beispielsweise in Ullmann's Encyclopedia of Industrial Chemistry, 2008 Edition.

Die Dosiermenge des Kristallisationshilfsmittels in die Mutterlauge entspricht bei kontinuierlichen Verfahren nach einer Phase der Aufkonzentration der in den abgetrennten Kristallen vorhandenen Menge an Mischungskomponente.

Nach der Herstellung der erfindungsgemäßen Mischkristalle können diesen wahlweise weitere Hilfsstoffe zugesetzt werden; beispielsweise bekannte Antibackmittel wie Maismehl, Magnesiumoxid und/oder Magnesiumcarbonat oder bekannte Trennmittel wie Salze von Fettsäuren, beispielsweise Stearinsäure, Calcium- und/oder Magnesiumstearat, Silikate, Siliziumdioxid, Talkum oder andere gebräuchliche Antibackmittel. Es ist jedoch ein Vorzug des erfindungsgemäßen Verfahrens, dass solche Zusätze im Gehalt stark reduziert oder sogar völlig auf den Einsatz verzichtet werden kann. Dosierungen von über 5000 ppm Magnesiumcarbonat sind im Allgemeinen nur bei extremen Lagertemperaturen und -zeiten von Vorteil.

Ferner betrifft die vorliegende Erfindung die Verwendung der erfindungsgemäßen Mischkristalle zur Herstellung von Backwaren, als Säureregulator in anderen Speisen, in Herstellung von Kosmetikprodukten, in der Synthese und Formulierung von Pharmaprodukten, sowie als Treibmittel in technischen Verfahren wie z. B. die Herstellung von Moosgummi oder für Feuerlöschformulierungen.

Ferner betrifft die vorliegende Erfindung ein Verfahren zur Herstellung von Backwaren, das dadurch gekennzeichnet ist, dass die erfindungsgemäßen Mischkristalle als modifiziertes Backtriebmittel eingesetzt werden.

Das Verfahren zur Herstellung von Backwaren wird ansonsten auf üblicher, dem Fachmann bekannter Weise durchgeführt.

Das Verfahren zur Herstellung von Backwaren ist folglich ferner dadurch gekennzeichnet, dass ein Teig zubereitet wird, der üblicherweise eine Stärkequelle wie Mehl und/oder Kartoffelstärke, eine Proteinquelle wie Eiweiß, häufig Fette wie Butter, Öl und/oder Margarine, und meist weitere Zutaten wie Zucker, Gewürze, Früchte oder ähnliches enthält..Die Zutaten werden wie üblich intensiv mechanisch vermischt, beispielsweise durch Rühren oder Kneten. Neben dem Backtriebmittel können weitere Zutaten verwendet werden, die ebenfalls zu Porosität in den erzeugten Backwaren führen, beispielsweise Hefe und/oder Sauerteig, ebenso kann die Porosität durch Einblasen von Gasen wie Luft in den Teig erhöht werden. Das Backtriebmittel kann wahlweise auch vor der eigentlichen Teigzubereitung mit einzelnen Komponenten des Teigs vorvermischt werden. Es kann ferner mit trockenen Komponenten des Teigs, beispielsweise Mehl, Zucker, Gewürzen, sonstigen Geschmacksstoffen und/oder Trockenei zu einer Backmischung vermischt werden, aus der durch Zugabe von Flüssigkeit ein Teig hergestellt und anschließend gebacken wird.

Die Menge des zugesetzten, Backtriebmittels mit modifizierter Kristallform wird so gewählt, dass die gewünschte Porosität eingestellt wird, dies ist mittels weniger Routineversuche leicht zu optimieren.

Üblicherweise wird die Menge an Backtriebmittel so gewählt, dass pro 100 g der eingesetzten Stärkequelle (z. B. Mehl und/oder Kartoffelstärke) vorteilhaft 1,5 bis 3,5 g Gase (Kohlendioxid, Ammoniak und/oder Wasserdampf), bevorzugt 2 bis 3 g Gase, insbesondere 2,35 bis 2,85 g Gase entwickelt werden.

Werden unporösere Backwaren hergestellt, ist die Menge entsprechend zu verringern; und für porösere entsprechend zu erhöhen.

Der Vorteil der vorliegenden Erfindung liegt darin, dass die erfindungsgemäßen Mischkristalle eine runde Form und glatte Oberflächen aufweisen. Somit bilden sich bei der Lagerung und bei der Anwendung der erfindungsgemäßen Mischkristalle als modifiziertes Backtriebmittel wesentlich weniger und darüber hinaus leichter wieder auflockerbare Agglomerate.

Da das Kristallisationshilfsmittel mit dem Backtriebmittel in einem Mischkristall vorliegt, kann es ferner zu keiner Zeit zu einer unerwünschten Separation führen.

Darüber hinaus wird das Ziel der Verhinderung von Verbackungen bereits mit einer sehr geringen Menge an Kristallisationshilfsmittel erreicht. Ferner ist kein zusätzlicher Verfahrensschritt zur Herstellung des Backtriebsmittels erforderlich.

Es lassen sich Backwaren einer gut steuerbaren Porosität herstellen.

### Beispiele

### 1. Kristallisation

Ammoniumbicarbonat (ABC), Wasser und Additiv wurden gemäß Tabelle 1 auf 40 °C unter Rühren erwärmt. Nach kompletter Lösung wurde schnell auf 0°C unter Rühren abgekühlt. Die entstandenen Kristalle wurden abgekühlt, getrocknet und unter dem Mikroskop mit einer Vergrößerung von 20:1 begutachtet.

**Tabelle 1:**

| Blindversuch | | | | |
|---|---|---|---|---|
| | ABC [g] | Wasser [g] | Additiv | |
| 1. (Vergleichsversuch) | 30,8 | 131,2 | 0 | Figur 2 |
| Pektin | | | | |
| | ABC [g] | Wasser [g] | Additiv [mg] | |
| 2. | 30,8 | 131,2 | 0,31 (10 ppm) | Figur 3 A |
| 3. | 30,8 | 131,2 | 1,5 (50 ppm) | Figur 3 B |
| Tylose | | | | |
| | ABC [g] | Wasser [g] | Additiv [mg] | |
| 4. | 30,8 | 131,2 | 0,15 (5 ppm) | Figur 4 A |
| 5. | 30,8 | 131,2 | 0,75 (25 ppm) | Figur 4 B |
| 6. (Vergleichsversuch, in Analogie zur EP 1 161 872) | 30,8 | 131,2 | 31 (1000 ppm) | Figur 4 C |
| Gelatine | | | | |
| | ABC [g] | Wasser [g] | Additiv [mg] | |
| 7. | 30,8 | 131,2 | 0,31 (10 ppm) | Figur 5 |

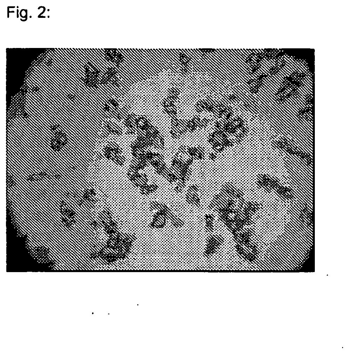

Die Ergebnisse sind in den Figuren 2 bis 5 dargestellt.

Aus Figur 2 und 4C ist deutlich die Agglomerationsneigung der Ammoniumbicarbonat-Kristalle zu erkennen. Die erfindungsgemäßen Mischkristalle (Figuren 3 A/B, 4 A/B und 5) hingegen weisen bei gleicher Vergrößerung nur einzelne Kristalle auf, die keine Agglomerationsneigung zeigen.

### 2. Penetrationstest

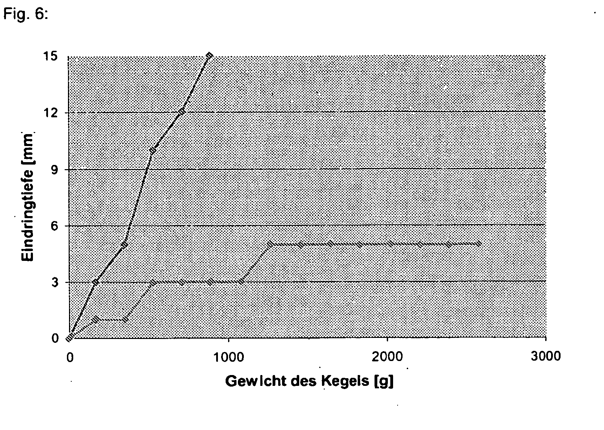

Probe 1: Mischkristalle aus ABC und 2 ppm Pektin, 3000 ppm Magnesimcarboant als Antibackmittel
Probe 2: ABC ohne Zugabe von Kristallisationshilfsmittel (Stand der Technik), 3000 ppm Magnesiumcarbonat als Antibackmittel
Beide Proben wurden 2 Wochen unter einem Lagerdruck von 1,25 kPa gelagert. Anschließend wurde das Penetrometer auf der Mitte des gelagerten Produkts gelegt und nach und nach mit höheren Gewichten belastet. Die Schichtdicke betrug 15 mm. Die Ergebnisse sind in der Tabelle 2 und in der Figur 6 dargestellt.

**Tabelle 2:**

| Gewicht des Kegels [g] | Probe 1 Eindringtiefe [mm] | Probe 2 Eindringtiefe [mm] |
|---|---|---|
| 0 | 0 | 0 |
| 170 | 3 | 1 |
| 350 | 5 | 1 |
| 531 | 10 | 3 |
| 713 | 12 | 3 |
| 896 | 15 | 3 |
| 1081 | | 3 |
| 1267 | | 5 |
| 1454 | | 5 |
| 1642 | | 5 |
| 1831 | | 5 |
| 2021 | | 5 |
| 2206 | | 5 |
| 2391 | | 5 |
| 2579 | | 5 |

Während bei der Probe 2 der Kegel nur schubweise (Plateaus in der Eindringtiefe-Gewichtskurve) und auch bei höherer Gewichtsbelastung nicht tief ins Produkt eindrang, sank der Kegel bei der Probe 1 nahezu proportional mit steigendem Gewicht in das Produkt, bis das Produkt vollständig verdrängt war.

Aus den Ergebnissen der Tabelle 2 geht hervor, dass die erfindungsgemäßen Mischkristalle (Probe 1) im Vergleich zum Stand der Technik (Probe 2) sehr gut rieselfähig sind und nicht einmal unter Druck ansatzweise Verbackungen aufweisen. Die Probe aus dem Stand der Technik (Probe 2) weist allerdings bereits bei geringem Druck Verbackungen auf.

### 3. Rieselfähigkeit nach Lagerung

Für den Lagerversuch wurden 25-kg-Kunststoffsäcke mit frisch produzierter Ware befüllt und mit Gewicht (Paletten mit Lagerware) belastet. Anschließend wurden die Säcke geöffnet und begutachtet. Die Lagerungsbedingungen sind in Tabelle 3.1 bis 3.3 zusammengefasst.

Der Verbackungsgrad von Ammoniumbicarbonat (ABC) wurde in einer Skala von 1 bis 5 eingestuft.

### Beurteilung:

1 = ABC ist klumpenfrei und gut rieselfähig
2 = ABC ist leicht auflockerbar und dann gut rieselfähig; es bleiben keine Klümpchen zurück
3 = ABC ist auflockerbar und dann rieselfähig; es bleiben Klümpchen zurück
4 = ABC ist schwer auflockerbar und dann bedingt rieselfähig; es bleiben größere Klümpchen zurück
5 = ABC nicht auflockerbar / nicht rieselfähig

Allen Proben der folgenden Tabellen wurden nach der Kristallisation zusätzlich die angegebenen Mengen von Magnesiumcarbonat als Antibackmittel zugesetzt.

**Tabelle 3.1:**

| | ABC nach Stand der Technik, 500 ppm MgCO₃ | Mischkristalle aus ABC und 2 ppm Pektin, 500 ppm MgCO₃ |
|---|---|---|
| Belastung: 1 Tonne | (Figur 7 A) | (Figur 7 B) |
| Lagerung: 1 Monat | | |
| Bewertung: | 4 | 1 |

**Tabelle 3.2:**

| | ABC nach Stand der Technik, 3000 ppm MgCO₃ | Mischkristalle aus ABC und 2 ppm Pektin, 3000 ppm MgCO₃ |
|---|---|---|
| Belastung: 2 Tonnen Lagerung: 5 Monate | (Figur 8 A) | (Figur 8 B) |
| Bewertung: | 5 | 2 |

**Tabelle 3.3:**

| | ABC nach Stand der Technik, 8000 ppm MgCO₃ | Mischkristalle aus ABC und 2 ppm Pektin, 8000 ppm MgCO₃ |
|---|---|---|
| Belastung: 2 Tonnen | (Figur 9 A) | (Figur 9 B) |
| Lagerung: 6 Monate | | |
| Bewertung: | 4 | 1 |

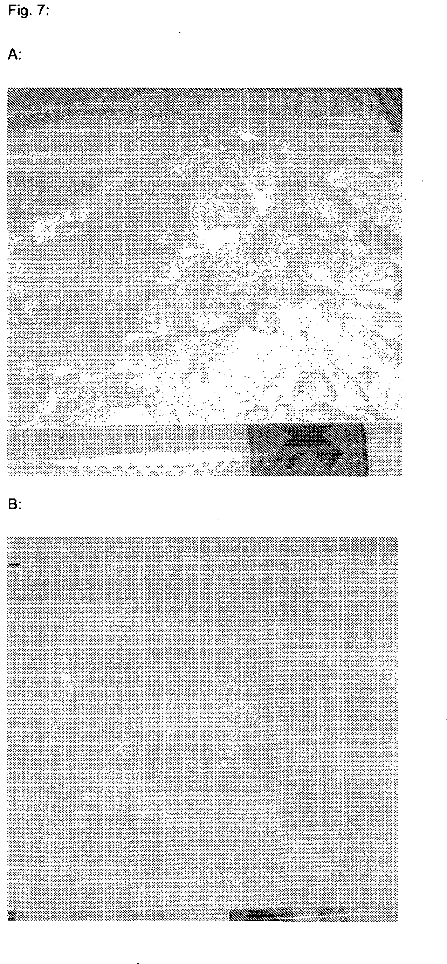

Figur 7: Während das ABC nach Stand der Technik stark verbacken ist und auch beim Bewegen des Pulvers größere Klumpen verbleiben, sind die erfindungsgemäßen Mischkristalle mit Pektin sehr gut rieselfähig und absolut klümpchenfrei.

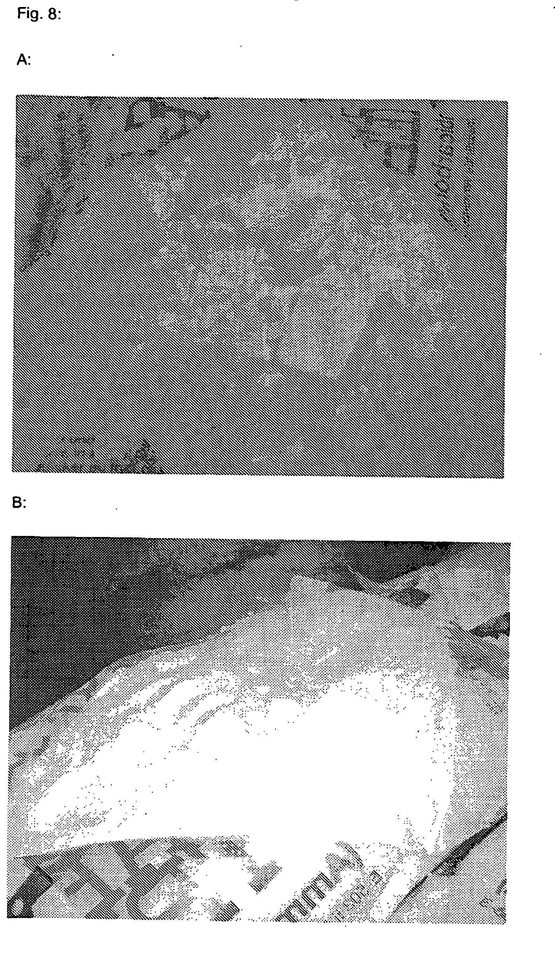

Figur 8: Während das ABC nach Stand der Technik sehr stark verbacken ist und selbst bei Aufstoßen des Sackes große, feste Klumpen übrigbleiben, sind die erfindungsgemäßen Mischkristalle mit Pektin gut rieselfähig. Die kleinen Klümpchen verschwinden beim Bewegen der Ware.

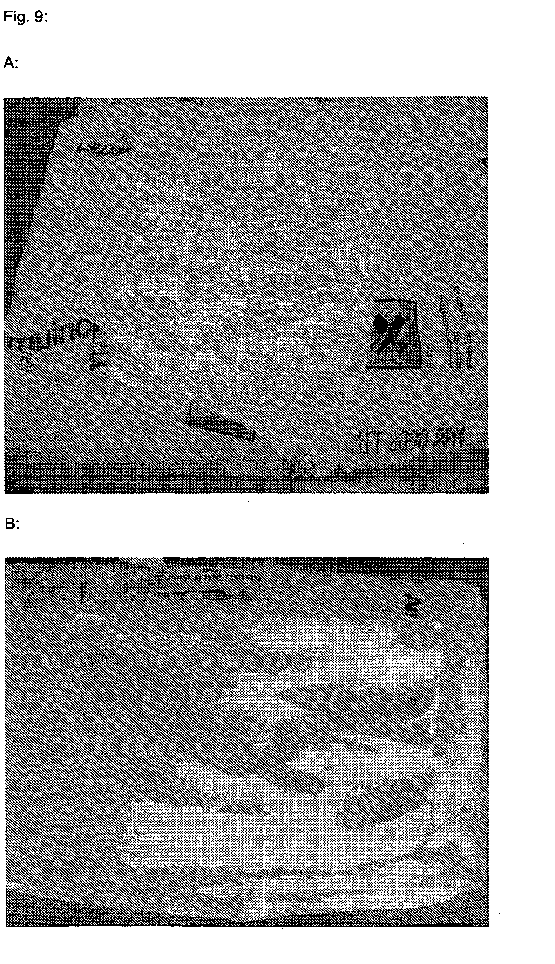

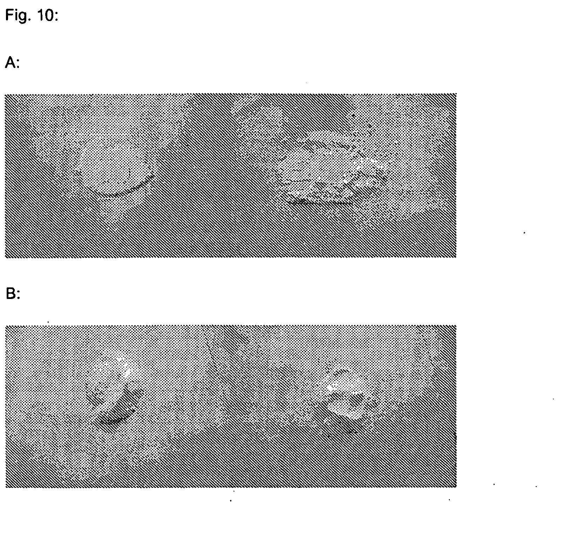

Figur 9: Während das ABC nach Stand der Technik stark verbacken ist, sind die Mischkristalle mit Pektin gut rieselfähig ohne die Bildung von Klümpchen.

### 4. Rieselfähigkeit nach Lagerung im Vergleich zu EP 1 161 872

Es wurde eine ca. 5 mm dicke Schicht aus ABC-Mischkristallen mit 25 ppm und 1000 ppm Tylose mit ca. 6 cm Durchmesser für 10 Minuten unter einem Gewicht von 13,75 kg gelagert (ca. 500 g/cm² Druck). Danach wurde das verdichtete Produkt durch Anheben des Papiers auf ein weiteres Papier umgefüllt und der Verbackungsgrad anhand der Skala aus Beispiel 3 beurteilt.

**Tabelle 4:**

| | ABC mit 1000 ppm Tylose (Vergleichsbeispiel in Analogie zur EP 1 161 872) | ABC mit 25 ppm Tylose |
|---|---|---|
| 10 Minuten bei 500 g/cm² Druck | (Figur 10 A) | (Figur 10 B) |
| Ergebnis: | 3 | 1 |

Anmerkung: Die Angabe "ppm" in den Beispielen steht für Gew.-ppm.

## Patentansprüche

1. Mischkristalle enthaltend
a) Backtriebmittel
b) 0,1 bis 5000 Gew.-ppm Kristallisationshilfsmittel, bezogen auf die Gesamtmenge des Backtriebmittels, in Form von mindestens einem Polymer, wobei beim Einsatz von hydrophilen Cellulosederivaten als Kristallisationshilfsmittel sich deren Menge auf kleiner 100 Gew.- ppm, bezogen auf die Gesamtmenge des Backtriebmittels, reduziert.

2. Mischkristalle nach Anspruch 1, wobei das Kristallisationshilfsmittel mindestens ein hydrophiles, kristallisationsbeeinflussendes Polymer beinhaltet.

3. Mischkristalle nach Anspruch 1 oder 2, wobei das Kristallisationshilfsmittel mindestens ein Polymer auf Basis von (Hetero)Polysacchariden und/oder (Poly)Peptiden beinhaltet.

4. Mischkristalle nach den Ansprüchen 1 bis 3, wobei die Komponente b) 0,5 bis 2000 Gew.-ppm Kristallisationshilfsmittel in Form von mindestens einem Polymer beinhaltet, wobei beim Einsatz von hydrophilen Cellulosederivaten als Kristallisationshilfsmittel sich deren Menge auf kleiner 80 Gew.- ppm reduziert.

5. Mischkristalle nach den Ansprüchen 1 bis 4, wobei die Komponente b) 1 bis 100 Gew.-ppm Kristallisationshilfsmittel in Form von mindestens einem Polymer beinhaltet, wobei beim Einsatz von hydrophilen Cellulosederivaten als Kristallisationshilfsmittel sich deren Menge auf 1 bis 50 Gew.- ppm reduziert.

6. Mischkristalle nach den Ansprüchen 1 bis 5, wobei als Komponente b) Polyacrylsäure oder deren Salze mit Ammonium, Natrium oder Kalium, Polymethacylsäure oder deren Salze mit Ammonium, Natrium oder Kalium, Acrylsäure oder deren Salze mit Ammonium, Natrium oder Kalium, MethacrylsäureCopolymere oder deren Salze Ammonium, Natrium oder Kalium, Acrylsäure-Maleinsäure-Copolymere oder deren Salze mit Ammonium, Natrium oder Kalium, Polyasparaginsäure oder deren Salze,
Polymine, Polyvinylamine und Copolymere mit Polyvinylalkohol und/oder Poly-dimethyl-allyl-ammoniumchlorid
Polyethylenglykole, Polypropylenglykole, Random und Blockpolymere auf Basis Ethylenoxid mit Alkylenoxiden
Methyl- Ethyl-, Propyl-, Carboximethyl- Hydroxiethyl-, Hydroxipropyl- Methylhydroxidethyl-, Methylhydroxipropyl-, Methylhydroxibutyl-, Ethylhydroxidethyl-, Carboximethylhydroxiethyl- und/oder Benzylcellulose
Alginsäure (E 400), Natriumalginat (E 401), Kaliumalginat (E 402), Ammoniumalginat (E 403), Calciumalginat (E 404), Propylenglycolalginat (E 405), Agar-Agar (E 406), Carrageen (E 407), Verarbeitete Eucheuma-Algen (E 407a), Johannisbrotkernmehl (E 410), Guarkernmehl (E 412), Traganth (E 413), Gummi arabicum (E414), Xanthan (E 415), Karaya (E 416), Tarakernmehl (E 417), Gellan (E 418), Komjak-Gummi/Konjak-glucomannan (E 425), Sojabohnen-Polyose (E426), Pektin/Amidiertes Pektin (E 440), Mikrokristalline Cellulose/cellulosepulver (E 460), Methylcellulose (E 461), Ethylcwellulose (E 462), Hydroxypropylcellulose (E 463), Hydroxypropylmethylcellulose (E 464), Ethylmethylcellulose (E 465), Carboxymethylcellulose/Natriumcarboxymethylcellulose (E 466), Vernetzte Natriumcarboxymethylcellulose (E 468), enzymatisch hydrolysierte Carboxymethylcellulose (E 469), Polydextrose (E 1200), Polyvinylpyrrolidon (E 1201), Polyvinylpolypyrrolidon (E 1202), Pullulan (E 1204), Oxidierte Stärke (E 1404), Monostärkephosphat (E 1410), Distärkephosphat (E 1412), Phosphatiertes Distärkephosphat (E 1413), Acetyliertes Distärkephosphat (E 1414), Acetylierte Stärke (E 1420), Acetylierte Distärkeadipat (E 1422), Hydroxypropylstärke (E 1440), Hydroxypropyldistärkephosphat (E 1442), Stärkenatriumoctenylsuccinat (E 1450), Acetylierte oxydierte Stärke (E 1451),
verwendet werden.

7. Mischkristalle nach den Ansprüchen 1 bis 6, wobei die Komponente b) ausgewählt ist aus der Gruppe bestehend aus Alginsäure (E 400), Natriumalginat (E 401), Kaliumalginat (E 402), Ammoniumalginat (E 403), Calciumalginat (E 404), Propylenglycolalginat (E 405), Pektin (E 440), amidiertes Pektin (E 440), Carrageen (E 407), Gellan (E 418), Gummi Arabicum (E414), Karaya (E 416), Traganth (E 413) und Xanthan (E 415).

8. Mischkristalle nach Anspruch 1, wobei als Komponente b) Pektin und/oder amidiertes Pektin (E 440) verwendet wird.

9. Mischkristalle nach den Ansprüchen 1 bis 8, wobei als Backtriebmittel Natriumcarbonat, Natriumhydrogencarbonat, Kaliumcarbonat, Kaliumhydrogencarbonat, Ammoniumcarbonat und/oder Ammoniumhydrogencarbonat verwendet wird.

10. Mischkristalle nach den Ansprüchen 1 bis 8, wobei als Backtriebmittel Ammoniumhydrogencarbonat verwendet wird.

11. Verfahren zur Herstellung der Mischkristalle nach den Ansprüchen 1 bis 10, **dadurch gekennzeichnet, dass** das Kristallisationshilfsmittel vor und/oder während des Kristallisationsschritts des Backtriebmittels zugegeben wird.

12. Verfahren zur Herstellung von Backwaren, **dadurch gekennzeichnet, dass** die Mischkristalle nach den Ansprüchen 1 bis 10 als modifiziertes Backtriebmittel eingesetzt werden.

13. Verwendung der Mischkristalle nach den Ansprüchen 1 bis 10 zur Herstellung von Backwaren, als Säureregulator in Speisen, in Herstellung von Kosmetikprodukten, in der Synthese und Formulierung von Pharmaprodukten, sowie als Treibmittel in technischen Verfahren.

## Claims

1. A mixed crystal comprising
a) baking raising agent
b) 0.1 to 5000 ppm by weight crystallization aid, based on the total amount of the baking raising agent, in the form of at least one polymer, wherein when hydrophilic cellulose derivatives are used as crystallization aid, the amount thereof is reduced to less than 100 ppm by weight, based on the total amount of the baking raising agent.

2. The mixed crystal according to claim 1, wherein the crystallization aid comprises at least one hydrophilic crystallization-influencing polymer.

3. The mixed crystal according to claim 1 or 2, wherein the crystallization aid comprises at least one polymer based on (hetero)polysaccharides and/or (poly)peptides.

4. The mixed crystal according to claims 1 to 3, wherein the component b) comprises 0.5 to 2000 ppm by weight of crystallization aid in the form of at least one polymer, wherein when hydrophilic cellulose derivatives are used as crystallization aid, the amount thereof is reduced to less than 80 ppm by weight.

5. The mixed crystal according to claims 1 to 4, wherein the component b) comprises 1 to 100 ppm by weight of crystallization aid in the form of at least one polymer, wherein when hydrophilic cellulose derivatives are used as crystallization aid, the amount thereof is reduced to 1 to 50 ppm by weight.

6. The mixed crystal according to claims 1 to 5, wherein, as component b), use is made of polyacrylic acid or salts thereof with ammonium, sodium or potassium,
polymethacrylic acid or salts thereof with ammonium, sodium or potassium, acrylic acid or salts thereof with ammonium, sodium or potassium, methacrylic acid copolymers or salts thereof with ammonium, sodium or potassium,
acrylic acid-maleic acid copolymers or salts thereof with ammonium, sodium or potassium, polyaspartic acid or salts thereof,
polymines, polyvinylamines and copolymers with polyvinyl alcohol and/or poly-dimethylallylammonium chloride
polyethylene glycols, polypropylene glycols, random and block polymers based on ethylene oxide with alkylene oxides
methyl-, ethyl-, propyl-, carboxymethyl-, hydroxyethyl-, hydroxypropyl-methylhydroxyethyl-, methylhydroxypropyl-, methylhydroxybutyl-, ethylhydroxyethyl-, carboximethylhydroxyethyland/or benzylcellulose
alginic acid (E 400), sodium alginate (E 401), potassium alginate (E 402), ammonium alginate (E 403), calcium alginate (E 404), propylene glycol alginate (E 405), agar-agar (E 406), carrageenan (E 407), processed Eucheuma algae (E 407a), carob bean meal (E 410), guar seed meal (E 412), tragacanth (E 413), gum arabic (E414), xanthan (E 415), karaya (E 416), tara gum (E 417), gellan (E 418), konjak gum/konjak glucomannan (E 425), soybean-polyose (E426), pectin/amidated pectin (E 440), microcrystalline cellulose/cellulose powder (E 460), methylcellulose (E 461), ethylcellulose (E 462), hydroxypropylcellulose (E 463), hydroxypropylmethylcellulose (E 464), ethylmethylcellulose (E 465), carboxymethylcellulose/sodium carboxymethylcellulose (E 466), crosslinked sodium carboxymethylcellulose (E 468), enzymatically hydrolyzed carboxymethylcellulose (E 469), polydextrose (E 1200), polyvinylpyrrolidone (E 1201), polyvinylpolypyrrolidone (E 1202), pullulan (E 1204), oxidized starch (E 1404), monostarch phosphate (E 1410), distarch phosphate (E 1412), phosphated distarch phosphate (E 1413), acetylated distarch phosphate (E 1414), acetylated starch (E 1420), acetylated distarch adipate (E 1422), hydroxypropylstarch (E 1440), hydroxypropyldistarch phosphate (E 1442), starch sodium octenyl succinate (E 1450), acetylated oxidized starch (E 1451).

7. The mixed crystal according to claims 1 to 6, wherein the component b) is selected from the group consisting of alginic acid (E 400), sodium alginate (E 401), potassium alginate (E 402), ammonium alginate (E 403), calcium alginate (E 404), propylene glycol alginate (E 405), pectin (E 440), amidated pectin (E 440), carrageenan (E 407), gellan (E 418), gum arabic (E414), karaya (E 416), tragacanth (E 413) and xanthan (E 415).

8. The mixed crystal according to claim 1, wherein, as component b), use is made of pectin and/or amidated pectin (E 440).

9. The mixed crystal according to claims 1 to 8, wherein, as baking raising agent, use is made of sodium carbonate, sodium hydrogencarbonate, potassium carbonate, potassium hydrogencarbonate, ammonium carbonate and/or ammonium hydrogencarbonate.

10. The mixed crystal according to claims 1 to 8, wherein, as baking raising agent, use is made of ammonium hydrogencarbonate.

11. A method of producing the mixed crystals according to claims 1 to 10, which comprises adding the crystallization aid before and/or during the crystallization of the baking raising agent.

12. A method of producing bakery products, which comprises using the mixed crystals according to claims 1 to 10 as modified baking raising agent.

13. The use of the mixed crystals according to claims 1 to 10 for producing bakery products, as acid regulator in foods, in production of cosmetics products, in the synthesis and formulation of pharmaceutical products, and also as raising agent in industrial processes.

## Revendications

1. Cristaux mixtes contenant
a) un agent de levée
b) de 0,1 à 5 000 ppm en poids d'adjuvant de cristallisation, par rapport à la quantité totale de l'agent de levée, sous forme d'au moins un polymère, dans le cas de l'utilisation de dérivés hydrophiles de cellulose en tant qu'adjuvants de cristallisation leur quantité étant réduite à moins de 100 ppm en poids, par rapport à la quantité totale de l'agent de levée.

2. Cristaux mixtes selon la revendication 1, dans lesquels l'adjuvant de cristallisation comporte au moins un polymère hydrophile, influant sur la cristallisation.

3. Cristaux mixtes selon la revendication 1 ou 2, dans lesquels l'adjuvant de cristallisation comporte au moins un polymère à base d'(hétéro)polysaccharides et/ou de (poly)peptides.

4. Cristaux mixtes selon les revendications 1 à 3, dans lesquels le composant b) comporte de 0,5 à 2 000 ppm en poids d'adjuvant de cristallisation sous forme d'au moins un polymère, dans le cas de l'utilisation de dérivés hydrophiles de cellulose en tant qu'adjuvants de cristallisation leur quantité étant réduite à moins de 80 ppm en poids.

5. Cristaux mixtes selon les revendications 1 à 4, dans lesquels le composant b) comporte de 1 à 100 ppm en poids d'adjuvant de cristallisation sous forme d'au moins un polymère, dans le cas de l'utilisation de dérivés hydrophiles de cellulose en tant qu'adjuvants de cristallisation leur quantité étant réduite à 1-50 ppm en poids.

6. Cristaux mixtes selon les revendications 1 à 5, dans lesquels on utilise comme composant b) un poly(acide acrylique) ou ses sels avec l'ammonium, le sodium ou le potassium, un poly(acide méthacrylique) ou ses sels avec l'ammonium, le sodium ou le potassium, l'acide acrylique ou ses sels avec l'ammonium, le sodium ou le potassium, des copolymères d'acide méthacrylique ou leurs sels d'ammonium, de sodium ou de potassium, des copolymères d'acide acrylique/acide maléique ou leurs sels avec l'ammonium, le sodium ou le potassium, le poly(acide aspartique) ou ses sels,
des polymines, des polyvinylamines et des copolymères avec le poly(alcool vinylique) et/ou le poly(chlorure de diméthyl-allylammonium)
des polyéthylèneglycols, des polypropylèneglycols, des polymères statistiques et séquencés à base d'oxyde d'éthylène avec des oxydes d'alkylène
la méthyl-, éthyl-, propyl-, carboxyméthyl-, hydroxyéthyl-, hydroxypropyl-, méthylhydroxyéthyl-, méthylhydroxypropyl-, méthylhydroxybutyl-, éthylhydroxyéthyl-, carboxyméthylhydroxyéthyl- et/ou benzylcellulose
l'acide alginique (E 400), l'alginate de sodium (E 401), l'alginate de potassium (E 402), l'alginate d'ammonium (E 403), l'alginate de calcium (E 404), l'alginate de propylèneglycol (E 405), l'agar-agar (E 406), le carraghénane (E 407), des algues Eucheuma transformées (E 407a), la farine de graines de caroube (E 410), la farine de graines de guar (E 412), la gomme adragante (E 413), la gomme arabique (E 414), la gomme xanthane (E 415), la gomme karaya (E 416), la farine de graines de tara (E 417), le gellane (E 418), la gomme konjac/le glucomannane de konjac (E 425), le polyose de soja (E426), la pectine/pectine amidée (E 440), la cellulose microcristalline/poudre de cellulose (E 460), la méthylcellulose (E 461), l'éthylcellulose (E 462), l'hydroxypropylcellulose (E 463), l'hydroxypropylméthylcellulose (E 464), l'éthylméthylcellulose (E 465), la carboxyméthylcellulose/carboxyméthylcellulose sodique (E 466), la carboxyméthylcellulose réticulée (E 468), la carboxyméthylcellulose hydrolysée par voie enzymatique (E 469), le polydextrose (E 1200), la polyvinylpyrrolidone (E 1201), la polyvinylpolypyrrolidone (E 1202), le pullulane (E 1204), l'amidon oxydé (E 1404), le phosphate de mono-amidon (E 1410), le phosphate de diamidon (E 1412), le phosphate de diamidon phosphaté (E 1413), le phosphate de diamidon acétylé (E 1414), l'amidon acétylé (E 1420), l'adipate de diamidon acétylé (E 1422), l'amidon hydroxypropylé (E 1440), le phosphate de diamidon hydroxypropylé (E 1442), l'octénylsuccinate d'amidon sodique (E 1450), l'amidon oxydé acétylé (E 1451).

7. Cristaux mixtes selon les revendications 1 à 6, dans lesquels le composant b) est choisi dans le groupe constitué par l'acide alginique (E 400), l'alginate de sodium (E 401), l'alginate de potassium (E 402), l'alginate d'ammonium (E 403), l'alginate de calcium (E 404), l'alginate de propylèneglycol (E 405), la pectine (E 440), la pectine amidée (E 440), le carraghénane (E 407), le gellane (E 418), la gomme arabique (E 414), la gomme karaya (E 416), la gomme adragante (E 413) et la gomme xanthane (E 415).

8. Cristaux mixtes selon la revendication 1, dans lesquels on utilise comme composant b) la pectine et/ou la pectine amidée (E 440).

9. Cristaux mixtes selon les revendications 1 à 8, dans lesquels on utilise comme agent de levée le carbonate de sodium, l'hydrogénocarbonate de sodium, le carbonate de potassium, l'hydrogénocarbonate de potassium, le carbonate d'ammonium et/ou l'hydrogénocarbonate d'ammonium.

10. Cristaux mixtes selon les revendications 1 à 8, dans lesquels on utilise comme agent de levée l'hydrogénocarbonate d'ammonium.

11. Procédé pour la préparation des cristaux mixtes selon les revendications 1 à 10, **caractérisé en ce qu'**on ajoute l'adjuvant de cristallisation avant et/ou pendant l'étape de cristallisation de l'agent de levée.

12. Procédé pour la fabrication de produits de boulangerie, **caractérisé en ce qu'**on utilise des cristaux mixtes selon les revendications 1 à 10 en tant qu'agent de levée modifié.

13. Utilisation des cristaux mixtes selon les revendications 1 à 10, pour la fabrication de produits de boulangerie, en tant que régulateur d'acide dans des aliments, dans la fabrication de produits cosmétiques, dans la synthèse et la formulation de produits pharmaceutiques, ainsi que comme agent d'expansion dans des procédés industriels.
